(19) Europäisches Patentamt / European Patent Office / Office européen des brevets

(11) **EP 3 462 879 B1**

(12) **FASCICULE DE BREVET EUROPEEN**

(45) Date de publication et mention
de la délivrance du brevet:
**04.08.2021 Bulletin 2021/31**

(21) Numéro de dépôt: **17727217.6**

(22) Date de dépôt: **02.06.2017**

(51) Int Cl.:
*A01N 63/20* (2020.01)   *A01N 25/04* (2006.01)
*A01P 21/00* (2006.01)

(86) Numéro de dépôt international:
**PCT/EP2017/063438**

(87) Numéro de publication internationale:
**WO 2017/207752 (07.12.2017 Gazette 2017/49)**

(54) **MÉTHODE POUR AMÉLIORER LE DÉVELOPPEMENT DES PLANTES**

VERFAHREN ZUR VERBESSERUNG DER ENTWICKLUNG VON PFLANZEN

METHOD FOR IMPROVING THE DEVELOPMENT OF PLANTS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.06.2016 FR 1655009**

(43) Date de publication de la demande:
**10.04.2019 Bulletin 2019/15**

(73) Titulaire: **Danstar Ferment AG
6300 Zug (CH)**

(72) Inventeurs:
• **WHITING, Mike
Saskatoon
Saskatchewan S7K 6E5 (CA)**
• **DELAUNOIS, Bertrand
31200 Toulouse (FR)**

(74) Mandataire: **IPAZ
16, rue Gaillon
75002 Paris (FR)**

(56) Documents cités:
WO-A1-2014/046553   WO-A1-2015/142185
WO-A1-2015/142186   WO-A1-2017/127535
WO-A2-03/057861   WO-A2-2006/071369
WO-A2-2017/019633   US-A- 5 503 652
US-A1- 2012 129 695   US-A1- 2014 109 249
US-A1- 2014 115 731

**Description**

**DOMAINE TECHNIQUE**

La présente demande concerne un procédé de préparation d'une souche de bactérie inactivée, d'une composition la comprenant et leur utilisation pour améliorer le développement des plantes.

**DESCRIPTION DE LA TECHNIQUE ANTÉRIEURE**

La recherche de nouvelles stratégies, visant à maintenir ou accroître la productivité des systèmes agricoles tout en diminuant les apports d'intrants chimiques, ont mis en évidence l'importance cruciale des microorganismes dans le fonctionnement biologique des agrosystèmes. Ces recherches ont conduit à l'émergence de la notion de «biofertilisants» et de «biostimulants», pouvant être définis comme des produits contenant par exemple des microorganismes vivants, inactivés et/ou extraits de microorganismes qui, lorsqu'ils sont appliqués au sol ou sur les plantes, occupent la rhizosphère, voire colonisent les tissus végétaux, et stimulent le développement de la plante en augmentant par exemple l'assimilation des nutriments et la production des phytohormones.

Le brevet US 5,589,381 décrit l'isolement d'un élément de contrôle biologique comprenant une souche de *Bacillus licheniformis,* qui contrôle la brûlure des semis due à *Fusarium* dans le maïs.

Le brevet US 5,503,652 décrit l'isolement de souches capables de promouvoir l'élongation des racines chez les plantes.

Le brevet US 5,935,839 décrit l'utilisation d'*Arthrobacter* sp. et de *Pseudomonas fluorescens* pour promouvoir la croissance des semis de conifères, dans lequel les rhizobactéries favorisant la croissance des plantes (Plant Growth Promoting Rhizobacteria PGPR) sont sélectionnées selon leur capacité à pousser dans des sols acides et froids typiques de conifères.

Le brevet US 5,503,651 décrit l'utilisation de souches PGPR qui favorisent la croissance des céréales, d'oléagineuses et de maïs en fonction des capacités chimiotactiques et en colonisant les racines des souches.

Le brevet US 5,496,547 enseigne l'isolement des mutants de *Pseudomonas* qui sont des agents de lutte biologique efficace contre *Rhizoctonia solani.*

Le brevet US 4,849,008 enseigne l'application de *Pseudomonas* sur les racines, les plantes, les graines, les fragments de pommes ou du sol, de plantes-racines pour améliorer le rendement des cultures racines.

Le brevet US 4,584,274 décrit des souches de *Pseudomonas* résistant aux bactériophages utiles pour promouvoir la croissance des plantes-racines.

La demande internationale WO 2003/057861 décrit l'isolement et l'identification d'un certain nombre de rhizobactéries favorisant la croissance des plantes (PGPR), qui oxydent le soufre en sulfate utile pour promouvoir la croissance des plantes, telles que RAY12, identifié comme *Achromobacter piechaudii* ; RAY28, identifié comme *Agrobacterium tumefaciens,* RAY132, identifié comme *Stenotrophomonas maltophilia* ; et RAY209, identifié comme *Delftia acidovorans.* Les bactéries vivantes sont inoculées dans le sol.

Le brevet US 6,194,193 décrit l'utilisation d'une formulation pour renforcer la croissance de la plante, qui comprend un mélange de souches de *Bacillus* et *Paenibacillus,* qui produisent des phytohormones.

La demande internationale WO 2006/071369 décrit une méthode permettant d'améliorer la survie et la stabilité des bactéries présentes dans un inoculant liquide pendant son stockage et lorsque les bactéries sont placées sur les semences. Le procédé décrit a pour objectif de maintenir les bactéries en vie afin qu'elles puissent coloniser la rhizosphère.

US 2012/129695 décrit une méthode de production d'un agent biostimulant des plantes comprenant la préparation d'un hydrolysat de cellules bactériennes.

US 5 503 652 décrit l'isolement dans le sol de souches bactériennes (notamment *Pseudomonas putida/fluorescens* ou *Serratia liquefaciens*) capables de coloniser les racines des plantes.

Les documents WO 2014/046553, WO 2015/142185, US 2014/109249, US 2014/115731 et WO 2015/142186 décrivent des méthodes de sélection dirigée de microorganismes utiles pour conférer des propriétés bénéfiques aux plantes. Les méthodes mises en œuvres dans ces documents reposent sur la présence de variabilité (telle que la variabilité génétique ou la variabilité du phénotype) dans les plantes et les populations microbiennes utilisées.

Un autre exemple d'effet hormonal connu est celui des *Azospirillum* spp (voir en particulier, Kucey (1988), Plant growth-altering effects of Azospirillum brasilense and Bacillus C-11-25 on two wheat cultivars. Journal of Applied Microbiology, volume 64, Issue 3, pages 187-196).

Certains biofertilisants sont composés d'organismes vivants, ils doivent donc être produits, formulés, et vendus de manière à ce que leur viabilité et leur activité biologique soient maintenues. Par ailleurs, le succès de l'inoculation microbienne pour la production agricole est grandement influencé par le nombre de cellules viables introduit dans le sol (Duquenne et al., 1999, FEMS Microbiology Ecology 29 : 331-339). La viabilité de l'inoculum est un facteur important pour le succès et la colonisation adéquate de la rhizosphère afin d'obtenir l'effet positif désiré sur la croissance des plantes. Un inconvénient majeur de l'utilisation des biofertilisants est que les conditions spécifiques de sol, de températures et d'humidité peuvent varier grandement d'un site à l'autre et que ces variations peuvent influencer la viabilité microbienne et par conséquent, le rendement et la croissance des plantes.

Il existe donc un besoin pour une composition permettant de stimuler le développement des plantes avec une efficacité améliorée sans avoir les inconvénients liés au maintien de la viabilité de ce type de biofertilisants/biostimulants.

## SOMMAIRE

La présente invention concerne l'utilisation d'une souche de bactérie inactivée du genre *Delftia,* ou une composition contenant ladite souche de bactérie inactivée, pour améliorer le développement des plantes, ladite souche de bactérie inactivée correspondant à des bactéries ou préparations de bactéries tuées par procédés physiques, biochimiques, chimiques ou physico-chimiques et présentant une viabilité inférieure à 50 %, ladite bactérie inactivée étant caractérisée en ce qu'elle a une efficacité biostimulante sur le développement des plantes supérieure à celle de la même souche de bactérie vivante ou de la composition la contenant.

La présente invention concerne également une méthode pour améliorer le développement des plantes, comprenant l'administration d'une souche de bactérie inactivée du genre *Delftia,* ou une composition la contenant, ladite souche de bactérie inactivée correspondant à des bactéries ou préparations de bactéries tuées par procédés physiques, biochimiques, chimiques ou physico-chimiques et présentant une viabilité inférieure à 50 %, ou d'une composition comprenant ladite souche de bactérie inactivée.

La présente divulgation concerne un procédé pour augmenter l'efficacité biostimulante d'une souche de bactérie vivante ou une composition la contenant, caractérisée en ce que le procédé comprend une étape d'inactivation de la souche de bactérie vivante, la souche de bactérie inactivée ainsi obtenue ayant une efficacité biostimulante sur le développement des plantes supérieure à celle obtenue avec la même souche de bactérie vivante ou avec une composition la contenant.

La présente divulgation concerne également une souche de bactérie inactivée pour améliorer le développement des plantes ou une composition la contenant, caractérisée en ce que la souche de bactérie inactivée permet une amélioration du développement des plantes par rapport à la même souche de bactérie vivante ou de la composition la contenant.

La présente divulgation concerne également une souche de bactérie inactivée pour améliorer le développement des plantes ou une composition la contenant, caractérisée en ce que la souche de bactérie inactivée possède une efficacité biostimulante sur le développement des plantes supérieures à celle obtenue avec la même souche de bactérie vivante ou avec une composition la contenant.

Avantageusement, la souche de bactérie inactivée ou la composition la contenant, obtenue selon le procédé de la présente divulgation ont une efficacité biostimulante sur le développement des plantes supérieures à celle obtenue avec la même souche de bactérie vivante ou avec une composition la contenant.

Avantageusement, l'utilisation de la composition et la méthode selon la présente invention permettent d'améliorer le développement des plantes sans avoir à considérer la viabilité d'un inoculum bactérien.

## BRÈVE DESCRIPTION DES FIGURES

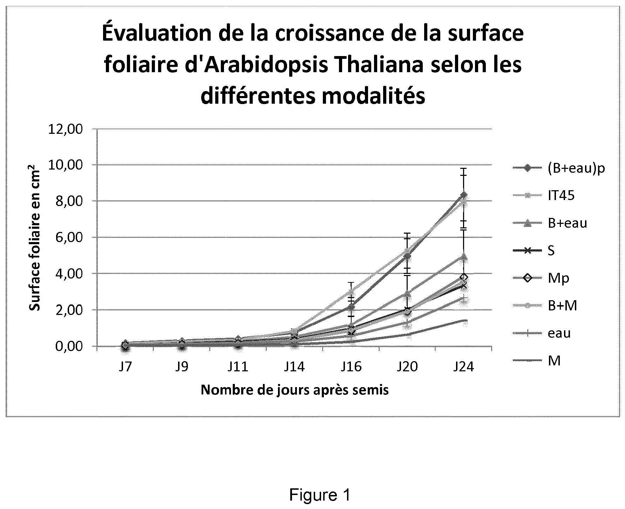

La Figure 1 illustre l'évaluation de la croissance de la surface foliaire *d'Arabidopsis thaliana* selon les modalités suivantes : M est le milieu de culture isolé de *Delftia acidovorans* RAY209; Eau est de l'eau sans bactéries actives ou inactives ou milieu de culture; B+M est *Delftia acidovorans* RAY209 dans son milieu de culture; Mp est le milieu de culture isolé de *Delftia acidovorans* RAY209 pasteurisé; S est une suspension de soufre; B + eau est *Delftia acidovorans* RAY209 dans l'eau; IT45 est un témoin positif (*Bacillus amyloliquefaciens* IT45); (B+eau)p est une solution pasteurisée de *Delftia acidovorans* RAY209 dans l'eau conforme à l'invention.

La Figure 2 illustre les poils absorbants racinaires des plants de canola *(Brassica napus* cultivar 5525CL) 36 jours après le traitement avec de l'eau stérile.

La Figure 3 illustre les poils absorbants racinaires des plants de canola *(Brassica napus* cultivar 5525CL) 36 jours après le traitement avec le milieu de culture de la souche de *Delftia acidovorans* RAY209.

La Figure 4 illustre les poils absorbants racinaires des plants de canola (Brassica napus cultivar 5525CL) 36 jours après le traitement avec la souche de *Delftia acidovorans* RAY209 inactivée par traitement à la presse de French (haute pression suivi d'une décompression rapide).

La Figure 5 illustre les poils absorbants racinaires des plants de canola *(Brassica napus* cultivar 5525CL) 36 jours après le traitement avec une souche de *Lactobacillus rhamnosus* inactivée par traitement à la presse de French (haute pression suivi d'une décompression rapide).

La Figure 6 illustre les poils absorbants racinaires des plants de canola *(Brassica napus* cultivar 5525CL) 36 jours après le traitement avec la souche de *Delftia acidovorans* RAY209 vivante.

La Figure 7 illustre les poils absorbants racinaires des plants de canola *(Brassica napus* cultivar 5525CL) 36 jours après le traitement avec une souche de *Lactobacillus rhamnosus* vivante.

La Figure 8 illustre les poils absorbants racinaires des plants de canola *(Brassica napus* cultivar 5525CL) 36 jours après le traitement avec la souche de *Delftia acidovorans* RAY209 inactivée par pasteurisation.

La Figure 9 illustre les poils absorbants racinaires des plants de canola *(Brassica napus* cultivar 5525CL) 36 jours après le traitement avec une souche de *Lactobacillus rhamnosus* inactivée par pasteurisation.

## DESCRIPTION DÉTAILLÉE

Le terme « bactéries vivantes » ou « souches de bactéries vivantes » correspond à des bactéries ou préparations de bactéries présentant une viabilité supérieure à 70 %.

Le terme « bactéries inactivées » ou « souches de bactéries inactivées » correspond à des bactéries ou préparations de bactéries tuées, par procédés physiques, biochimiques, chimiques ou physico-chimiques et présentant une viabilité inférieure à 50 %.

Le terme « biomasse » correspond à l'ensemble de la matière, organique et minérale, constituant un organisme.

Le terme « biostimulant(e) » correspond à la stimulation du développement des plantes. Par exemple, le développement des plantes peut inclure un des paramètres suivants : l'enracinement, la surface foliaire, la floraison, la fructification, la hauteur des plantes, la biomasse, la germination, le rendement de la récolte.

Le terme « rhizobactéries favorisant la croissance des plantes » ou « PGPR» correspond aux bactéries de la rhizosphère bénéfiques à la croissance et à la santé des plantes.

Le terme « milieu de culture » correspond à un support contenant les éléments nécessaires à la croissance bactérienne, qui permet la culture des bactéries utilisées selon l'invention. Selon un mode de réalisation, le milieu de culture peut contenir les bactéries utilisées selon l'invention durant leur croissance ou être un milieu de culture exempt des bactéries utilisées selon la présente invention, si celles-ci sont séparées de leur milieu par un procédé, mettant en œuvre notamment mais non exclusivement une étape de filtration ou une étape de centrifugation. Selon un mode réalisation, le milieu de culture est de préférence un milieu liquide. Tous ces milieux ainsi que les procédés de fermentation usuels sont bien connus de l'homme du métier.

Le terme « support de culture » correspond à un ensemble de produits destiné à servir de milieu de culture à certains végétaux. Leur mise en œuvre aboutit à la formation de milieux possédant une porosité en air et en eau, telle qu'ils sont capables à la fois d'ancrer les organes absorbants des plantes et de leur permettre d'être en contact avec les solutions nécessaires à leur croissance. Ils sont généralement composés de matières organiques et/ou de matières inorganiques. Ils sont généralement composés de tourbe, d'autres matériaux organiques (notamment fibres de coco, écorces, fibres de bois, composts) et de matériaux inorganiques (notamment terres, sables, pouzzolane, argiles, laines minérales, perlite, vermiculite).

Selon un premier aspect, la présente invention concerne l'utilisation d'une souche de bactérie inactivée du genre *Delftia,* ou une composition contenant ladite souche de bactérie inactivée, pour améliorer le développement des plantes, ladite souche de bactérie inactivée correspondant à des bactéries ou préparations de bactéries tuées par procédés physiques, biochimiques, chimiques ou physico-chimiques et présentant une viabilité inférieure à 50 %, ladite bactérie inactivée étant caractérisée en ce qu'elle a une efficacité biostimulante sur le développement des plantes supérieure à celle de la même souche de bactérie vivante ou de la composition la contenant.

Selon un second aspect, la présente invention concerne une méthode pour améliorer le développement des plantes, comprenant l'administration d'une souche de bactérie inactivée du genre *Delftia,* ou une composition la contenant, ladite souche de bactérie inactivée correspondant à des bactéries ou préparations de bactéries tuées par procédés physiques, biochimiques, chimiques ou physico-chimiques et présentant une viabilité inférieure à 50 %, ou d'une composition comprenant ladite souche de bactérie inactivée. Selon un mode de réalisation, la méthode comprenant l'administration d'une composition ou d'une souche de bactérie inactivée selon l'invention permet une amélioration du développement des plantes, supérieure à celle de la même composition contenant la même souche de bactérie vivante ou de la même souche de bactérie vivante.

Dans un mode de réalisation avantageux, l'étape d'inactivation mise en œuvre dans le procédé selon la divulgation est réalisée par des procédés physiques, biochimiques, chimiques ou physico-chimiques. Dans un mode de réalisation plus avantageux, la souche de bactérie vivante est inactivée par traitement thermique ou par traitement à hautes pressions. Avantageusement, la souche de bactérie vivante est inactivée par pasteurisation. Encore plus avantageusement, la souche de bactérie vivante est inactivée sans son milieu de culture.

Dans un mode de réalisation particulièrement avantageux, la souche de bactérie utilisée dans une utilisation ou dans une méthode selon l'invention est du genre *Delftia*Plus avantageusement, la souche de bactérie est la souche *Delftia acidovorans* ,. Dans un mode de réalisation encore plus avantageux, la souche de bactérie inactivée est la souche de *Delftia acidovorans* RAY209 déposée à l'ATCC le 25 avril 2002 sous le n° PTA-4249.

La présente divulgation concerne une souche de bactérie inactivée, ou une composition pour améliorer le développement des plantes, caractérisée en ce qu'elle comprend au moins une souche de bactérie inactivée. Avantageusement, la présente divulgation concerne une souche de bactérie inactivée pour améliorer le développement des plantes caractérisée en ce que la souche de bactérie inactivée permet une amélioration du développement des plantes par rapport à la même souche de bactérie vivante.

La présente divulgation concerne une souche de bactérie inactivée ou une composition pour améliorer le développement des plantes, caractérisée en ce qu'elle comprend au moins une souche de bactérie inactivée. Avantageusement, la présente divulgation concerne une souche de bactérie inactivée pour améliorer le développement des plantes caractérisée en ce que la souche de bactérie inactivée possède une efficacité biostimulante sur le développement des plantes supérieure à celle obtenue avec la même souche de bactérie vivante ou avec une composition la contenant.

La souche de bactérie utilisé selon l'invention peut provenir de toutes les espèces de bactéries du genre *Delftia.* Selon un mode de réalisation avantageux, les bactéries utilisées selon l'invention peuvent provenir de l'espèce *Delftia acidovorans,.* Plus avantageusement, la souche de bactérie utilisé est *Delftia acidovorans* RAY209 déposé au « American Type Culture Collection (ATCC) le 25 avril 2002 sous le n° PTA-4249 en accord avec le traité de Budapest sur la reconnaissance internationale du dépôt des micro-organismes aux fins de la procédure en matière de brevets.

La souche de bactérie utilisé dans le cadre de la présente invention est une rhizobactérie favorisant le développement des plantes ou « PGPR » du genre *Delftia.* Plus particulièrement, la souche de bactérie utilisé selon l'invention est de la souche *Delftia acidovorans* RAY209 déposée au « American Type Culture Collection (ATCC) le 25 avril 2002 sous le n° PTA-4249 en accord avec le traité de Budapest sur la reconnaissance internationale du dépôt des micro-organismes aux fins de la procédure en matière de brevets.

Selon un mode de réalisation avantageux, dans une utilisation ou un procédé selon l'invention, les bactéries sont inactivées notamment par procédé physique, chimique, biochimique ou physico-chimique. Selon un mode de réalisation, les bactéries sont inactivées par traitement par hautes pressions (par exemple au moyen d'une presse de French ou autres méthodes connues dans l'art). Dans un autre mode de réalisation, les bactéries de sont inactivées par traitement thermique. Plus avantageusement, les bactéries sont inactivées par pasteurisation. Selon un mode de réalisation particulièrement avantageux, la pasteurisation est effectuée en chauffant les bactéries vivantes à une température entre 60°C et 90°C, 62°C et 88°C, 65°C et 85°C, 75°C et 85°C, ou 80°C et 85°C. Selon un autre mode de réalisation, les bactéries sont pasteurisées sans leur milieu de culture. Selon un autre mode de réalisation, les bactéries sont inactivées avec leur milieu de culture. Selon un autre mode de réalisation, les bactéries sont inactivées sans leur milieu de culture.

Selon un mode de réalisation, l'amélioration du développement et/ou de la croissance et de la productivité des plantes et/ou l'augmentation de l'efficacité biostimulante sur le développement des plantes inclut notamment mais non exclusivement d'améliorer un des paramètres suivants : l'enracinement, la surface foliaire, le développement racinaire, la floraison, la fructification, la hauteur des plantes, la biomasse, la germination, le rendement de la récolte notamment en quantité, en qualité ou en précocité. Selon un mode de réalisation, l'augmentation de l'efficacité biostimulante sur le développement des plantes inclut la surface foliaire, le nombre des poils absorbants racinaires et/ou la hauteur des plantes.

Selon un mode de réalisation, la présente invention est applicable à tout type de plantes, notamment mais non exclusivement aux cultures céréalières (blé, orge, avoine, seigle, triticale), aux plantes sarclées (betterave sucrière, pomme de terre, mais), aux légumineuses (luzerne, trèfle, sainfoin), aux culture fourragères (ray-grass, fétuque, dactyle, festulolium, luzerne, vesce, navette, radis fourragés), aux oléoprotéagineuses (soja, canola, colza, pois, féverole, lupin blanc, tournesol), aux cultures légumières et maraîchères, à l'arboriculture fruitière, à la viticulture et aux cultures ornementales (production florales, gazons, pépinières de jeune plants).

Selon un mode de réalisation avantageux, la présente invention concerne une utilisation d'une souche de bactérie inactivée du genre *Delftia* ou d'une composition le comprenant, et une méthode pour améliorer le développement des plantes comprenant l'administration d'une souche de bactérie inactivée, caractérisées en ce que ladite composition comprend au moins une souche de bactérie inactivée, la souche de bactérie inactivée possédant une efficacité biostimulante sur le développement des plantes supérieure à celle de la même souche de bactérie vivante

Selon un mode de réalisation avantageux, dans une utilisation ou une méthode selon l'invention, la composition comprend de $10^4$ UFC/ml à $10^{12}$ UFC/ml, de $10^5$ UFC/ml à $10^{12}$ UFC/ml de $10^6$ UFC/ml à $10^{12}$ UFC/ml, de $10^7$ UFC/ml à $10^{12}$ UFC/ml, de $10^6$ UFC/ml à $10^{11}$ UFC/ml, de $10^6$ UFC/ml à $10^{10}$ UFC/ml, de $10^6$ UFC/ml à $10^9$ UFC/ml, ou de $10^6$ UFC/ml à $10^8$ UFC/ml de bactéries avant inactivation.

Selon un mode de réalisation, dans une utilisation ou une méthode selon l'invention, la composition comprend au moins une souche de bactérie inactivée à 100%, à 99 % (avec 1% d'au moins une souche de bactérie active), à 98% (avec 2% d'au moins une souche de bactérie active), à 97% (avec 3% d'au moins une souche de bactérie active), à 96% (avec 4% d'au moins une souche de bactérie active), à 95% (avec 5% d'au moins une souche de bactérie active), à 94% (avec 6% d'au moins une souche de bactérie active), à 93% (avec 7% d'au moins une souche de bactérie active), à 92% (avec 8% d'au moins une souche de bactérie active), à 91% (avec 9% d'au moins une souche de bactérie active), entre 90% et 85% (avec entre 10 et 15% d'au moins une souche de bactérie active), entre 85% et 80% (avec entre 15 et 20% d'au moins une souche de bactérie active), entre 80% et 75% (avec entre 20 et 25% d'au moins une souche de bactérie active), entre 75% et 70% (avec entre 25 et 30% d'au moins une souche de bactérie active), entre 70% et 65% (avec entre 30 et 35% d'au moins une souche de bactérie active), entre 65% et 60% (avec entre 35% et 40% d'au moins une souche de bactérie active), entre 60% et 55% (avec entre 40 et 45% d'au moins une souche de bactérie active) ou entre 55% et 50 % (avec entre 45% et 50% d'au moins une souche de bactérie active).

Selon un mode de réalisation particulièrement avantageux, dans une utilisation ou une méthode selon l'invention, la composition comprend au moins une souche de bactérie inactivée et un véhicule compatible pour l'agriculture. Plus particulièrement, le véhicule compatible pour l'agriculture est approprié pour l'administration de la bactérie inactivée sur la plante et/ou sur le sol. Selon un mode de réalisation, le véhicule est sous forme solide et/ou liquide. Selon un autre mode de réalisation, le véhicule est de l'eau. Selon un autre mode de réalisation, le véhicule est un mélange eau-herbicide. Selon un autre mode de réalisation, le véhicule est un mélange eau-fertilisant. Selon un autre mode de réalisation, le véhicule est un milieu de culture. Selon un mode de réalisation particulièrement avantageux, dans une utilisation ou une méthode selon l'invention, la composition comprend une souche de bactérie inactivée isolée de son milieu de culture.

Selon un mode de réalisation avantageux, dans une utilisation ou une méthode selon l'invention, la composition comprenant au moins une souche de bactérie inactivée pourra se présenter sous forme de poudre, de granules, de micro-granules, de traitement de semences, de formulations liquides, d'encapsulation des bactéries ou de suspensions liquides. Plus particulièrement, la composition est sous forme liquide.

Selon un mode de réalisation avantageux, dans une utilisation ou une méthode selon l'invention, la composition est en combinaison avec une formulation appropriée comprenant des poudres, notamment des poudres mouillables, des granulés, des micro-granulés, des traitements de semences, des encapsulations de bactéries, des formulations liquides, notamment mais non exclusivement des suspensions, dans l'eau, dans un solvant ou dans un milieu de culture.

Selon un mode de réalisation avantageux, dans une utilisation ou une méthode selon l'invention, la composition est sous une forme appropriée pour le traitement au sol, le traitement de la partie racinaire de la plante, le traitement foliaire de la plante, le traitement de la partie florale de la plante, le traitement de la partie fructifère de plante et/ou le traitement de la graine. Selon un autre mode de réalisation avantageux, la composition est administrée simultanément ou successivement, par application au sol, par trempage de racine, par traitement de semences ou par incorporation et/ou enrobage à un support de cultures, pelliculage avec des produits phytosanitaires ou d'engrais ou tout autre véhicule ou par tout moyen permettant la mise en contact immédiate ou future de la composition avec les semences ou les plantes à inoculer. Plus particulièrement l'application au sol est réalisée notamment mais non exclusivement par pulvérisation, épandage, arrosage, traitement au sol, ferti-irrigation, goutte à goutte, dans la raie de semis ou en plein. Selon un mode de réalisation avantageux, la composition comprend la souche de bactérie inactivée en combinaison avec d'autres microorganismes vivants, inactivés ou en extraits, tels que les bactéries, les champignons et/ ou les levures. Plus particulièrement, la composition comprend la souche de bactérie inactivée en combinaison avec d'autres souches de bactéries inactivées, lesdites bactéries favorisant le développement, la nutrition et la protection des plantes.

Selon un mode de réalisation avantageux, la composition comporte en outre des engrais, herbicides, insecticides, fongicides, bactéricides, solutions minérales et/ou supports de culture. Selon un autre mode de réalisation avantageux, la composition comporte en outre un substrat. Plus particulièrement, le substrat comprend des matières organiques, notamment mais non exclusivement de la tourbe, des matières inorganiques, notamment mais non exclusivement de la terre et/ou du sable et/ou de l'argile et/ou d'autres composants du sol et/ou des substances synthétiques. Plus particulièrement, la substance synthétique peut être un matériau absorbant comme par exemple un matériau granulé.

Selon un mode de réalisation, la composition contenant une souche de bactérie inactivée ou la souche de bactérie inactivée permet une amélioration du développement des plantes supérieure à celle obtenue avec la même composition contenant la même souche de bactérie vivante ou la même souche de bactérie vivante.

Selon un autre aspect, la présente invention concerne une plante obtenue en utilisant la composition, selon l'invention, ou en utilisant la souche de bactérie inactivée, selon l'invention, pour améliorer le développement d'une plante.

Selon un autre aspect, la présente invention concerne une plante obtenue en utilisant une méthode pour améliorer le développement d'une plante comprenant l'administration d'une composition ou d'une souche de bactérie inactivée, selon l'invention.

**EXEMPLES**

La présente demande se comprendra mieux à la lecture des exemples suivants qui sont donnés pour illustrer la demande et non à limiter la portée.

Des essais ont été effectués pour vérifier l'impact de certaines souches et essentiellement *Delftia acidovorans,* vivantes ou tuées, en présence ou non de leur milieu de culture, sur le développement d'une plante modèle.

**Exemple 1**

1-1/ Préparation des inocula de la souche bactérienne : *Delftia acidovorans*

La concentration bactérienne souhaitée dans chaque inocula a été estimé à un équivalent de $4.10^{11}$ UFC/m$^3$ avec une souche commerciale de *Delftia acidovorans,* référencée LPC8. Les pots utilisés lors des essais ayant un volume de 0,0003 m$^3$, il a été déterminé qu'il faudrait introduire $1,2.10^8$ UFC par pot.

1-2/ Technique de dénombrement des suspensions bactériennes :

La souche bactérienne *Delftia acidovorans* RAY209 est commercialisée dans une formule liquide contenant la suspension de bactéries dans son milieu de culture (BioBoost Liquid ou BBL). Afin de connaître la concentration bactérienne du BBL, un dénombrement des bactéries dans le produit a été effectué. À la suspension bactérienne (BBL) du Bag-in-Box™ initial a été prélevé 1 ml de solution afin d'être placé dans un tube à essai. De l'eau peptonée (9 mL) a été ajoutée dans le tube à essai contenant 1 ml de suspension bactérienne afin de faire une dilution au $10^{-1}$. Un second prélèvement de 1 ml de cette dilution a été mis dans un tube à essai et auquel a été ajouté 9 ml d'eau peptonée afin de faire une dilution au $10^{-2}$. Le procédé a été ensuite répété de façon identique jusqu'à obtenir une dilution au $10^{-4}$ et $10^{-5}$. À chacune de ces dilutions $10^{-4}$ et $10^{-5}$ a été prélevé 100 $\mu$l afin d'ensemencer la surface d'une boite de Pétri (milieu TSA : trypto-caséine soja). Un total de 6 boîtes de Pétri ont été ensemencées par dilution et l'incubation a été réalisée durant 48h à 30°C (ATCC., 2015 ; Larcher., 2015). Les colonies à la surface des boîtes ont été comptées et la concentration dans le BBL (produit commercial) a été estimée à $2.10^7$ UFC/ml.

1-3 Technique de dénombrement des suspensions bactériennes :

La souche bactérienne *Delftia acidovorans* RAY209 est commercialisée dans une formule liquide contenant la suspension de bactéries dans son milieu de culture Préparation de l'inoculum bactérie dans milieu de culture : « bactérie + surnageant » (B+M):
La suspension bactérienne BBL a été utilisée telle quelle pour la modalité « bactérie + surnageant » (B+M).

1-4/ Préparation de l'inoculum : « bactérie +eau » :

1400 ml de suspension bactérienne BBL ont été centrifugés à 8500 rotations par minutes (13000 g) pendant 15 minutes. Le culot bactérien a été dissout dans 700 ml d'eau et a été centrifugé à nouveau. Cette étape a été répétée 3 fois afin d'éliminer tout reste de surnageant de culture. Le culot a ensuite été re-suspendu dans de l'eau du robinet (environ 700ml). La concentration bactérienne a été à nouveau mesurée dans cette solution selon le protocole décrit à l'exemple 1-2. Après comptage des colonies à la surface des boîtes, la concentration des bactéries a été estimée à $4.10^7$ UFC/ml. Cette suspension en l'état correspond à la modalité « bactérie +eau » ;

1-5/ Préparation de la modalité « bactérie + eau, pasteurisé » ((B+eau)p) :

Environ 200 ml de la suspension « bactérie + eau » décrite à l'exemple 1-2 a été chauffée au bain marie pendant 20 minutes à 80°C afin d'obtenir la suspension « bactérie + eau, Pasteurisée » ((B+eau)p).

1-6/ Préparation de la modalité « surnageant de culture » (M) :

La suspension bactérienne BBL a été centrifugée à 8500 rotations par minutes (13000 g) pendant 15 minutes et environ 600 ml de surnageant a été prélevé pour l'inoculation des plantes. Ce prélèvement correspond à la modalité « milieu de culture » (M).

1-7/ Préparation de la modalité « Surnageant de culture pasteurisé » (Mp) :

La suspension bactérienne BBL a été centrifugée et 200 ml de surnageant de culture ont été récupérés après la centrifugation afin d'être chauffés au bain-marie pendant 20 minutes à 80°C. La solution résultante correspond à la modalité « Surnageant de culture pasteurisé » (Mp)

1-8/ Préparation de la modalité « soufre » (S) :

Dans un contenant a été mélangé 1g de soufre dans 100 mL d'eau pour obtenir la modalité « soufre » (S).

1-9/ Préparation de la modalité du témoin positif *Bacillus amyloliquefaciens* IT45 (IT45) :

Une préparation microbienne atomisée de *Bacillus amyloliquefaciens_IT45,* sous forme de poudre et concentrée à $2.10^{10}$ UFC/g, a été re-suspendue dans de l'eau pour obtenir la concentration cible de $4.10^7$ UFC/ml.

**Exemple 2**

2-1/ Préparation des plantes (*Arabidopsis thaliana*)

La croissance des plantes a été réalisée sur un milieu solide dans des conditions non-stériles afin de se rapprocher des conditions naturelles au champ selon les répétitions suivantes :

$$\text{Répétition biologique} = 8 \text{ modalités} \times 3 \text{ répétitions} \times 6 \text{ itérations} = 144 \text{ plantes}$$

2-2/ Préparation du semis

Les graines ont été conservées à 4°C afin d'assurer leur préservation ainsi qu'une germination correcte et synchronisée (ABRC, 2015). Environ 300-400 graines ont été placées de façon très clairsemée dans 3 boites de Pétri (14 cm de diamètre) sur du papier à germination. L'équivalent de 5 ml d'eau a été ajouté de façon à simplement imbiber le papier à germination. Les boîtes de Pétri ont été placées 3 jours au réfrigérateur (4°C) pour assurer la stratification des graines.

2-3/ Préparation des micro-serres et des pots

La croissance des plantes a été réalisée dans des pots (6 x 6 x 7 cm) placés dans des micro-serres (22 x 16 x 18 cm) par nombre de 6. Les pots ont été remplis de 150 g d'un mélange terreau-sable (2/3 de terreau et 1/3 de sable, m/m).
Les micro-serres ont été recouvertes d'irrinappe à l'intérieur afin de maintenir un environnement humide pour les plantes.
Les graines ont été ensuite prélevées des boîtes de Pétri et ont été placés sur les pots de terre (5-6 graines par pot) à l'aide d'une pince. Une seule pousse par pot a été gardée une fois que les graines ont germé.

2-4/ Inoculation et croissance des plantes

Chaque plantule a été inoculée au moment du semis avec les solutions/suspensions décrites à l'exemple 1 et comme ci-après. Les plantules ont été soumises à un cycle nychtéméral de 16h de jour à 23°C suivi de 8h de nuit à 18°C. Les pots ont été arrosés une à deux fois par jour durant la partie diurne du cycle. L'hygrométrie n'a pas été régulée et a été d'environ 70-80%. Chaque modalité a été inoculée selon les doses suivantes :

- A) *Delftia acidovorans* RAY209 dans son milieu de culture (B+M): 6ml/pot (18 pots)
- B) « bactérie + eau » (B+eau): 3ml/pot (18 pots)
- C) « (bactérie + eau) pasteurisée » (B+eau)p: 3ml/pot (18 pots)
- D) « surnageant de culture pasteurisé » (Mp): 6ml/pot (18 pots)
- E) eau : 3ml/pot (18 pots)
- F) « surnageant de culture » (M): 6ml/pot (18 pots)
- G) Suspension de *Bacillus amyloliquefaciens* IT45 : 1 ml/pot (18 pots)
- H) Soufre (S): 0,01 g/pot (18 pots) (1 mL par plante d'une solution à 1% (m/v)).

Les différents lots de pots ont été randomisés de manière à ce que les essais soient répartis de façon plus homogène dans la zone utilisée afin de s'affranchir au maximum des disparités des variables (température, luminosité, aération, humidité...) présentes dans la serre et la chambre de culture. 3 blocs de 8 mini-serres ont été mis en place dans la chambre de culture, avec une rotation intra-bloc.

2-5/ Mesures et analyses

La croissance des plantes a été évaluée par le développement de la surface des feuilles au cours du temps. Les plantes ont été prises en photos de haut tous les 2 jours et chaque photo a été analysée grâce au logiciel Fiji afin de pouvoir calculer la surface foliaire de chaque plante tout au long du cycle.

Les données des mesures ont été répertoriées dans un fichier Excel (surface foliaire, masse sèche, masse fraîche) et ces données ont été analysées grâce au logiciel XIstat. Le test de Tukey (test post-hoc de comparaisons multiples) a été utilisé afin de conclure sur les différences significatives entre les moyennes des modalités (voir figure 1).

Tableau 1. Moyennes des surfaces foliaires pour chaque modalité et par jour

|  | J7 | J9 | J11 | J14 | J16 | J20 | J24 |
|---|---|---|---|---|---|---|---|
| (B+eau)p | 0,179 a | 0,302 a | 0,398 a | 0,742 ab | 2,166 ab | 4,960 a | 8,339 a |
| IT45 | 0,126 ab | 0,242 ab | 0,314 a | 0,837 a | 2,998 a | 5,263 a | 7,961 a |
| B+eau | 0,070 bc | 0,149 abc | 0,254 ab | 0,498 abc | 1,167 ab | 2,917 a | 4,975 a |
| S | 0,044 c | 0,125 bc | 0,226 ab | 0,360 abc | 0,964 ab | 2,021 a | 3,326 a |
| Mp | 0,050 c | 0,120 bc | 0,164 ab | 0,315 abc | 0,859 ab | 1,932 a | 3,809 a |
| B+M | 0,056 c | 0,106 bc | 0,158 ab | 0,296 abc | 0,890 ab | 1,913 a | 3,538 a |
| eau | 0,053 c | 0,101 bc | 0,173 ab | 0,252 bc | 0,553 ab | 1,312 a | 2,646 a |
| M | 0,018 c | 0,033 c | 0,050 b | 0,098 c | 0,218 b | 0,624 a | 1,414 a |
| Pr > F | 0,000 | 0,001 | 0,010 | 0,004 | 0,025 | 0,033 | 0,036 |
| Significatif (>95%) | Oui | Oui | Oui | Oui | Oui | Oui | Oui |

*a, b et c correspondant à des groupes homogènes de plantes traitées, utilisés pour la réalisation du test statistique de Tukey.*
*Pr>F : valeur seuil du degré de significativité*

Les différences constatées sur les mesures des surfaces foliaires de chaque modalité sont significatives (voir Tableau 1). Une différence significative entre le témoin positif (*Bacillus amyloliquefaciens* IT45) et le témoin négatif (eau) a été observée. De plus, la modalité (B+eau)p a montré les meilleurs résultats en terme de surface foliaire des plantes (supérieur au témoin positif) en comparaison des autres modalités. Le surnageant de culture M a présenté quant à lui la croissance foliaire la plus faible.

## Exemple 3

**Étude portant sur l'effet de l'inactivation de souches de *Delftia* et *Lactobacillus* sur les paramètres de croissance de plantules de canola**

L'objectif de cette étude est de déterminer l'effet d'un traitement de pasteurisation de souches de *Delftia acidovorans* et *Lactobacillus rhamnosus* sur la croissance de plantules de canola (*Brassica napus* cultivar 5525CL). Plus particulièrement, cette étude vise à comparer l'effet de la pasteurisation et de la non-pasteurisation (c'est-à-dire d'une culture bactérienne vivante) d'une souche bactérienne sur les paramètres de croissance de plantules de canola.

3-1/ Cultivar de canola

Des semences de canola *Brassica napus* appartenant au cultivar 5525CL (Brett Young) ont été désinfectées selon le protocole décrit dans Asaduzzaman et al. ("Metabolomics Differentiation of Canola Genotypes: Toward an Understanding of Canola Allelochemicals." Frontiers in Plant Science, vol. 5, 2015.doi:10.3389/fpls.2014.00765). Après séchage, les graines ont été mises à germer dans des boîtes de Pétri contenant de l'agar (15 g/l). Les graines germées sont transférées dans des poches de germination (Mega International), à raison de 2 graines/poche, contenant un mélange de terreau supplémenté d'une demi-dose d'une solution d'Hoagland No. 2 (Sigma, H2395).

3-2/ Modalités à l'étude

Tableau 2. Description des différentes modalités étudiées dans cet exemple.

| Modalité no. | Modalités |
|---|---|
| 1 | Suspension de *Delftia acidovorans* RAY 209 vivante (cellules lavées dans eau distillée stérile) |
| 2 | Suspension de *Delftia acidovorans* RAY 209 (cellules lavées dans eau distillée stérile) inactivée par pasteurisation |
| 3 | Suspension de *Delftia acidovorans* RAY 209 (cellules lavées dans eau distillée stérile) inactivée par traitement à la presse de French (haute pression suivi d'une décompression rapide) |
| 4 | Suspension de *Lactobacillus rhamnosus* R0011 vivante (cellules lavées dans eau distillée stérile) |
| 5 | Suspension de *Lactobacillus rhamnosus* R0011 (cellules lavées dans eau distillée stérile)) inactivée par pasteurisation |
| 6 | Suspension de *Lactobacillus rhamnosus* R0011 (cellules lavées dans eau distillée stérile) inactivée par traitement à la presse de French (haute pression suivi d'une décompression rapide) |
| 7 | Surnageant de culture de *Delftia acidovorans* RAY 209 (traitement sans bactéries) |
| 8 | Eau distillée stérile |

3-3/ Préparation des inoculants non-pasteurisés

Les souches bactériennes utilisées pour l'inoculation des semences de canola sont *Delftia acidovorans* RAY 209 (BBL) et *Lactobacillus rhamnosus* RO011 (Institut Rosell-Lallemand. Montréal, Qc, Canada). Les concentrations des solutions mères de bactéries sont, respectivement, de 3,71 E+08 cfu/ml et 2,21 E+11 cfu/ml.

Les solutions mères (1 ml) sont centrifugées à 8500 rpm pendant 15 minutes. Le culot est resuspendu dans 1 ml d'eau distillée stérile. Cette étape de lavage des cellules bactériennes est répétée 2 fois. Suite à la première centrifugation de la solution mère de *Delftia acidovorans* RAY 209, le surnageant est conservé pour le traitement futur des semences de canola (modalité 3). Les suspensions bactériennes dans l'eau distillée stérile sont conservées pour l'inoculation des semences de canola germées (modalités 1 et 4).

3-4/ Préparation des inoculants inactivés Deux techniques d'inactivation ont été testées : (1) pasteurisation ou traitement par la chaleur et (2) traitement à la presse de French (haute pression suivi d'une décompression rapide).

*(1) Traitement par la chaleur*

Pour chacune des 2 souches bactériennes étudiées, 1 ml de suspension bactérienne à une concentration de 3,71E+08 cfu/ml dans l'eau distillée stérile est transférée dans un tube Eppendorf (de 1,5 ml) et incubée à 80ºC dans un bain-marie pendant 20 minutes. Les suspensions bactériennes pasteurisées sont conservées pour l'inoculation des semences de canola germées (modalités 2 et 5).

*(2) Traitement à la presse de French (haute pression suivi d'une décompression rapide)*

Pour chacune des 2 souches bactériennes étudiées, 5 ml de suspension bactérienne à une concentration de 2,21E+11 cfu/ml dans l'eau distillée stérile sont soumis à un passage à la presse de French (American Instrument CO Inc.) à la pression de 18000 psi à 4ºC suivi d'une détente instantanée. Les cellules bactériennes lysées sont récupérées pour l'inoculation des semences de canola germées (modalités 3 et 6).

3-5/ Inoculation bactérienne des semences de canola

L'inoculation des semences de canola germées a été effectuée à l'aide d'une pipette et 10 $\mu$l des préparations obtenues (voir Tableau 2 pour la description des 8 modalités étudiées) ont été appliquées sur chaque semence de canola. Le Tableau 3 présente les concentrations bactériennes appliquées sur les semences de canola. Les semences traitées ont été maintenues sous atmosphère contrôlée dans une chambre de croissance avec 16 heures de lumière à 22 ºC et 8 heures d'obscurité à 18ºC.

Le dispositif expérimental comportait, pour chacune des modalités, 10 répétitions de 2 semences germées/poche de germination. Un total de 20 semences germées a donc été traité par modalité.

Tableau 3. Calculs des concentrations bactériennes appliquées par semence de canola

| Inoculant | Concentration de la solution mère | Dilution de la solution mère à 108 CFU/mL | Titre requis (CFU/ml) | Quantité appliquée/ semence ($\mu$l) | Titre finale (CFU/ semence (10 $\mu$l)) | Quantité d'inoculant dans le volume total ($\mu$L) | Quantité d'eau dans le volume total ($\mu$L) |
|---|---|---|---|---|---|---|---|
| D. acidovorans RAY 209 | 3,71E+08 | 3,71E+08 | 1,00E+08 | 10 | 1,00E+06 | 2,694 | 7,31 |
| L. rhamnosus R0011 | 2,21E+11 | 2,21E+08 | 1,00E+08 | 10 | 1,00E+06 | 4,525 | 5,48 |

3-6/ Notation des résultats

La prise de données a été effectuée après 8 jours, 22 jours et 36 jours suivant l'inoculation des semences de canola. Après 8 jours d'incubation, les racines latérales sont dénombrées. Après 22 jours d'incubation, 10 plantules par modalité sont récoltées. Dix plantules par modalité sont aussi récoltées après 36 jours d'incubation. Suite à la récolte des plantules de canola, la hauteur des pousses a été mesurée du collet à la plus haute feuille. Les racines ont été séparées du sol et mesurées. La biomasse végétale ainsi que la biomasse racinaire (pousses et racines séparément) ont aussi été mesurées à l'aide d'une balance de précision. Les poids secs des pousses et des racines ont été déterminées après séchage à 35ºC pendant 48 heures.

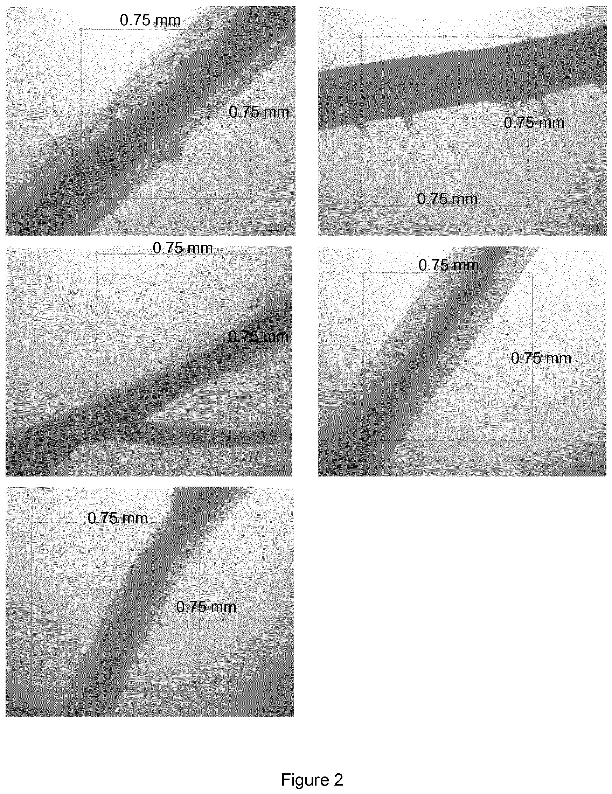

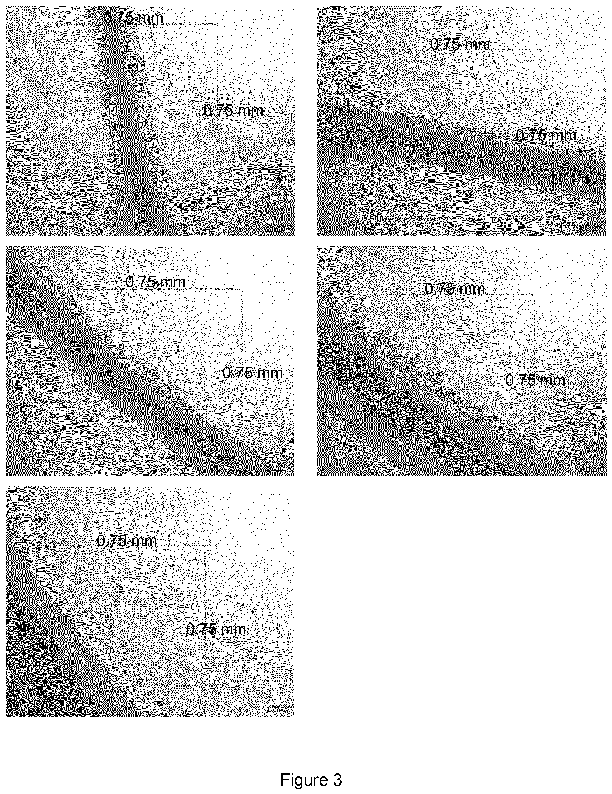

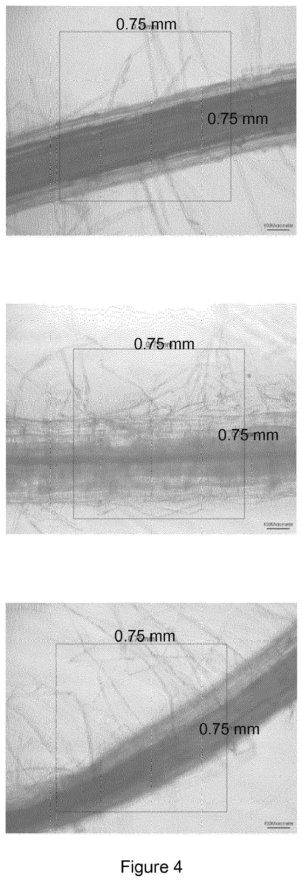

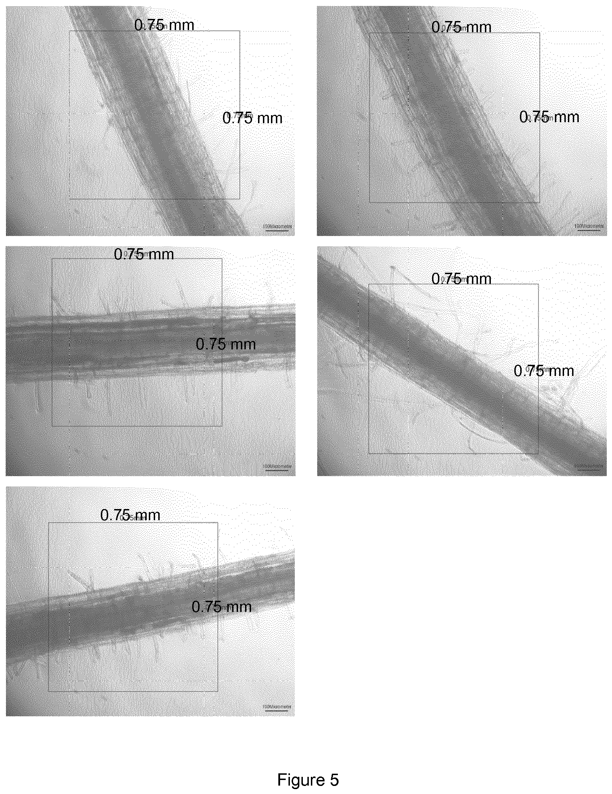

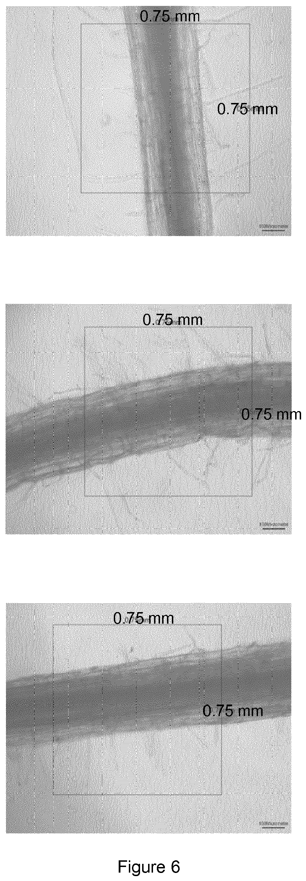

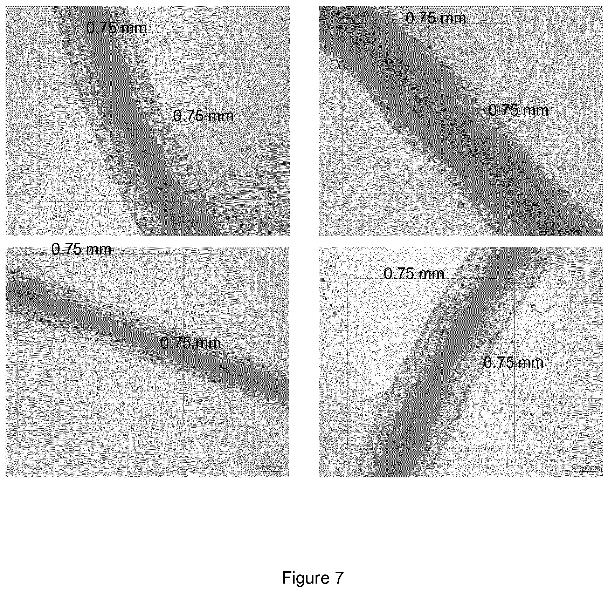

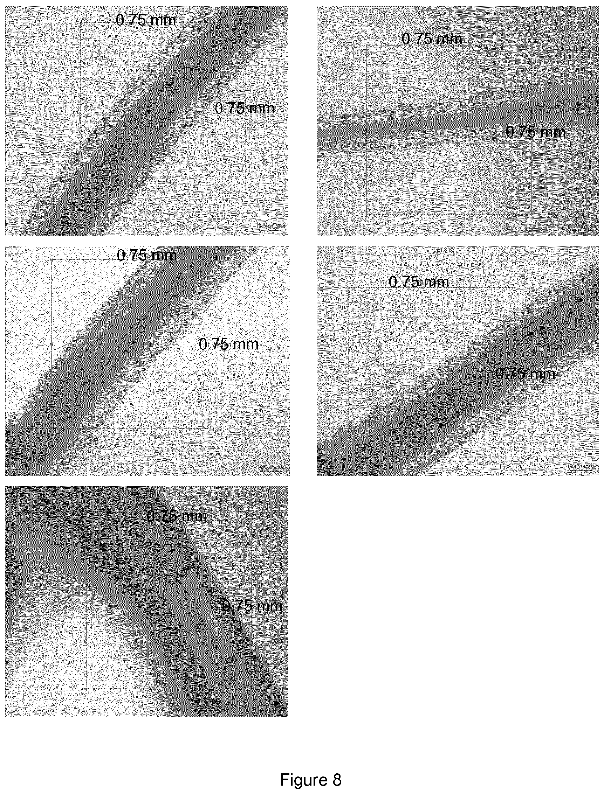

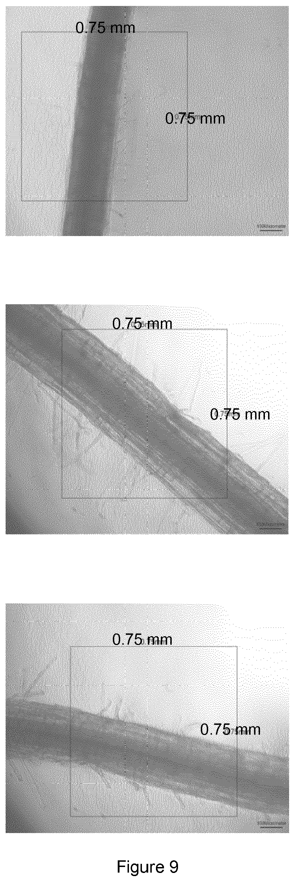

De plus, pour chacune des modalités, des observations microscopiques des poils absorbants racinaires ont été effectuées afin d'évaluer leur présence sur les racines des plantules de canola. Pour ce faire, pour chacune des modalités, 1 cm de racine primaire est placée dans une boîte de Pétri. La racine a été immergée d'eau afin que les poils absorbants racinaires soient en suspension. Les observations microscopiques ont été réalisées à un grossissement de 100X. Les poils racinaires ont été dénombrés sur une superficie de 0,75 mm$^2$. Les Figures 2 à 9 montrent les images des observations microscopiques des poils absorbants racinaires selon les modalités.

Des analyses de variance (ANOVA) à un facteur ont été réalisées ainsi que des tests de comparaison de variance de Bartlett afin de conclure quelles modalités présentent une variance différente des autres pour la hauteur des pousses (à l'aide du logiciel KyPlot). Le résultat de l'analyse sur la hauteur des pousses est présenté dans le Tableau 4.

Les résultats présentés démontrent que la souche de *Delftia acidovorans* est capable de stimuler le développement de la partie racinaire des plantes en augmentant le nombre de poils absorbants racinaires. Ceci aura, entre autres, comme effet d'améliorer l'assimilation des nutriments par les plantes.

Tableau 4. Comparaison de la hauteur des pousses de canola 36 jours après traitement.

| Traitement | Eau Stérile | Delftia vivante |
|---|---|---|
| Eau stérile | X | X |
| Milieu de culture de *Delfia* | + | X |
| *Lactobacillus* inactivée par pression | - | X |
| *Lactobacillus* pasteurisé | + | X |
| *Lactobacillus* vivant | - | X |

(suite)

| Traitement | Eau Stérile | Delftia vivante |
|---|---|---|
| Delftia vivante | - | X |
| Delftia inactivée par pression | + | ++ |
| Delftia pasteurisé | +++ | +++ |

Les symboles représentent la significativité des résultats issus de la comparaison des traitements indiqués en colonne par rapport aux traitements indiqués en ligne (test statistique d'analyse de variance ANOVA).

Légende

| symbole | signification |
|---|---|
| "+" | différence positive non significative |
| "-" | différence négative non significative |
| "=" | pas de différences |
| "++" | différence positive significative à P=0,1 |
| "--" | différence négative significative à P=0,1 |
| "+++" | différence positive significative à P=0,05 |
| "---" | différence négative significative à P=0,05 |

**Revendications**

1. Utilisation d'une souche de bactérie inactivée, ou d'une composition comprenant ladite souche de bactérie inactivée, pour améliorer le développement des plantes, ladite souche de bactérie inactivée correspondant à des bactéries ou préparations de bactéries tuées par procédés physiques, biochimiques, chimiques ou physico-chimiques et présentant une viabilité inférieure à 50%, ladite bactérie inactivée étant **caractérisée en ce que** :

   - elle a une efficacité biostimulante sur le développement des plantes supérieure à celle de la même souche de bactérie vivante, et
   - elle est du genre *Delftia.*

2. Utilisation selon la revendication 1, ladite bactérie inactivée étant de l'espèce *Delftia acidovorans.*

3. Utilisation selon la revendication 1 ou 2, ladite bactérie inactivée étant la souche de bactérie *Delftia acidovorans* RAY209 déposée à l'ATCC le 25 avril 2002 sous le n° PTA-4249.

4. Méthode pour améliorer le développement d'une plante comprenant l'administration d'une souche de bactérie inactivée du genre *Delftia,* ou d'une composition comprenant ladite souche de bactérie inactivée, ladite souche de bactérie inactivée correspondant à des bactéries ou préparations de bactéries tuées par procédés physiques, biochimiques, chimiques ou physico-chimiques et présentant une viabilité inférieure à 50%, ou d'une composition comprenant ladite souche de bactérie inactivée.

5. Méthode selon la revendication 4, **caractérisée en ce que** ladite souche de bactérie inactivée provient de l'espèce *Delftia acidovorans.*

6. Méthode selon la revendication 4 ou 5, **caractérisée en ce que** ladite souche de bactérie inactivée est la souche de bactérie *Delftia acidovorans* RAY209 déposée à l'ATCC le 25 avril 2002 sous le n° PTA-4249.

7. Méthode selon l'une quelconque des revendications 4 à 6, **caractérisée en ce que** ladite souche de bactérie inactivée est obtenue par traitement thermique.

8. Méthode selon l'une quelconque des revendications 4 à 7, **caractérisée en ce que** qu'elle comprend l'administration d'une composition comprenant au moins ladite souche de bactérie inactivée et un véhicule compatible pour l'agriculture.

9. Méthode selon l'une quelconque des revendications 4 à 8, **caractérisée en ce que** qu'elle comprend l'administration d'une composition comprenant au moins ladite souche de bactérie inactivée isolée de son milieu de culture.

10. Méthode selon l'une quelconque des revendications 4 à 9, **caractérisée en ce que** qu'elle comprend l'administration d'une composition comprenant au moins ladite souche de bactérie inactivée en combinaison avec d'autres microorganismes vivants, inactivés ou en extraits, tels que les bactéries, les champignons et/ ou les levures.

11. Méthode selon l'une quelconque des revendications 4 à 10, **caractérisée en ce que** qu'elle comprend l'administration d'une composition comprenant au moins ladite souche de bactérie inactivée en combinaison avec d'autres souches de bactéries inactivées.

12. Méthode selon l'une quelconque des revendications 4 à 11, **caractérisée en ce qu'**elle comprend l'administration d'une composition comprenant au moins ladite souche de bactérie inactivée et des engrais, herbicides, insecticides, fongicides, solutions minérales et/ou supports de culture.

13. Méthode selon l'une quelconque des revendications 4 à 12, **caractérisée en ce que** ladite composition se présente sous une forme appropriée pour le traitement au sol, le traitement de la partie racinaire de la plante, le traitement foliaire de la plante, le traitement des parties florales, le traitement des parties fructifères et/ou le traitement de la graine.

14. Méthode selon l'une quelconque des revendications 4 à 13, **caractérisée en ce que** ladite composition se présente sous forme de poudre, de granules, de micro-granules, de traitement de semences, de formulations liquides, d'encapsulation des bactéries ou de suspensions liquides.

## Patentansprüche

1. Verwendung eines inaktivierten Bakterienstamms oder einer Zusammensetzung, die den inaktivierten Bakterienstamm umfasst, zur Verbesserung der Entwicklung von Pflanzen, wobei der inaktivierte Bakterienstamm Bakterien oder Präparaten von Bakterien entspricht, die durch physikalische, biochemische, chemische oder physikalisch-chemische Verfahren abgetötet wurden und eine Lebensfähigkeit von unter 50% aufweisen, wobei das inaktivierte Bakterium **dadurch gekennzeichnet ist, dass**

   - es eine biostimulierende Wirksamkeit auf die Entwicklung von Pflanzen aufweist, die größer ist als die desselben, aber lebenden Bakterienstammes; und
   - es zur Gattung *Delftia* gehört.

2. Verwendung gemäß Anspruch 1, wobei das inaktivierte Bakterium zur Spezies *Delftia acidovorans* gehört.

3. Verwendung gemäß Anspruch 1 oder 2, wobei das inaktivierte Bakterium zum Bakterienstamm *Delftia acidovorans* RAY209 gehört, der am 25. April 2002 unter der Nr. PTA-4249 bei der ATCC hinterlegt wurde.

4. Verfahren zur Verbesserung der Entwicklung einer Pflanze, umfassend die Verabreichung eines inaktivierten Bakterienstamms der Gattung *Delftia* oder einer Zusammensetzung, die den inaktivierten Bakterienstamm umfasst, wobei der inaktivierte Bakterienstamm Bakterien oder Präparaten von Bakterien entspricht, die durch physikalische, biochemische, chemische oder physikalisch-chemische Verfahren abgetötet wurden und eine Lebensfähigkeit von unter 50% aufweisen, oder einer Zusammensetzung, die den inaktivierten Bakterienstamm umfasst.

5. Verfahren gemäß Anspruch 4, **dadurch gekennzeichnet, dass** der inaktivierte Bakterienstamm von der Spezies *Delftia acidovorans* stammt.

6. Verfahren gemäß Anspruch 4 oder 5, **dadurch gekennzeichnet, dass** es sich bei dem inaktivierten Bakterienstamm um den Bakterienstamm *Delftia acidovorans* RAY209 handelt, der am 25. April 2002 unter der Nr. PTA-4249 bei der ATCC hinterlegt wurde.

7.  Verfahren gemäß einem der Ansprüche 4 bis 6, **dadurch gekennzeichnet, dass** der inaktivierte Bakterienstamm durch Wärmebehandlung erhalten ist.

8.  Verfahren gemäß einem der Ansprüche 4 bis 7, **dadurch gekennzeichnet, dass** es die Verabreichung einer Zusammensetzung umfasst, die wenigstens den inaktivierten Bakterienstamm und einen mit der Landwirtschaft verträglichen Träger umfasst.

9.  Verfahren gemäß einem der Ansprüche 4 bis 8, **dadurch gekennzeichnet, dass** es die Verabreichung einer Zusammensetzung umfasst, die wenigstens den inaktivierten, aus seinem Kulturmedium isolierten Bakterienstamm umfasst.

10. Verfahren gemäß einem der Ansprüche 4 bis 9, **dadurch gekennzeichnet, dass** es die Verabreichung einer Zusammensetzung umfasst, die wenigstens den inaktivierten Bakterienstamm in Kombination mit anderen, lebenden, inaktivierten oder als Extrakte vorliegenden Mikroorganismen, wie Bakterien, Pilze und/oder Hefen, umfasst.

11. Verfahren gemäß einem der Ansprüche 4 bis 10, **dadurch gekennzeichnet, dass** es die Verabreichung einer Zusammensetzung umfasst, die wenigstens den inaktivierten Bakterienstamm in Kombination mit anderen inaktivierten Bakterienstämmen umfasst.

12. Verfahren gemäß einem der Ansprüche 4 bis 11, **dadurch gekennzeichnet, dass** es die Verabreichung einer Zusammensetzung umfasst, die wenigstens den inaktivierten Bakterienstamm und Dünger, Herbizide, Insektizide, Fungizide, Minerallösungen und/oder Kulturträger umfasst.

13. Verfahren gemäß einem der Ansprüche 4 bis 12, **dadurch gekennzeichnet, dass** die Zusammensetzung in einer Form vorliegt, die für die Behandlung am Boden, die Behandlung des Wurzelbereichs der Pflanze, die Blattbehandlung der Pflanze, die Behandlung der Blütenteile, die Behandlung der fruchttragenden Teile und/oder die Behandlung des Samens geeignet ist.

14. Verfahren gemäß einem der Ansprüche 4 bis 13, **dadurch gekennzeichnet, dass** die Zusammensetzung in Form eines Pulvers, Granulats, Mikrogranulats, einer Saatbehandlung, von flüssigen Zubereitungen, eingekapselten Bakterien oder flüssigen Suspensionen vorliegt.

**Claims**

1.  Use of an inactivated bacteria strain, or of a composition comprising said inactivated bacteria strain, to improve plant development, said inactivated bacteria strain corresponding to bacteria or bacteria preparations killed by physical, biochemical, chemical or physicochemical processes and having a viability less than 50%, said inactivated bacteria being **characterized in that**:

    - it has a greater biostimulant efficacy on plant development than that obtained with the same strain of living bacteria, and
    - it is of the genus *Delftia.*

2.  The use according to claim 1, said inactivated bacteria being of the species *Delftia acidovarans.*

3.  The use according to claim 1 or 2, said inactivated bacteria being the bacteria strain *Delftia acidovorans* RAY209 filed with the ATCC on April 25, 2002 under no. PTA-4249.

4.  A method to improve plant development comprising the administration of an inactivated bacteria strain of the genus *Delftia* or of a composition comprising said inactivated bacteria strain, said inactivated bacteria strain corresponding to bacteria or bacteria preparations killed by physical, biochemical, chemical or physicochemical processes and having a viability less than 50%, or to a composition comprising said inactivated bacteria strain.

5.  The method of claim 4, **characterized in that** said strain of inactivated bacteria originates from the species *Delftia acidovarans.*

6.  The method according to claim 4 or 5, **characterized in that** said inactivated bacteria strain is the bacteria strain *Delftia acidovorans* RAY209 filed with the ATCC on April 25, 2002 under no. PTA-4249.

7. The method according to anyone of claims 4 to 6, **characterized in that** said inactivated bacteria strain is inactivated by thermal treatment.

8. The method according to anyone of claims 4 to 7, **characterized in that** it comprises the administration of a composition comprising at least said inactivated bacteria strain and a vehicle compatible for agriculture.

9. The method according to anyone of claims 4 to 8, **characterized in that** it comprises the administration of a composition comprising at least said inactivated bacteria strain isolated from its culture medium.

10. The method according to anyone of claims 4 to 9, **characterized in that** it comprises the administration of a composition comprising at least said inactivated bacteria strain in combination with other living microorganisms, inactivated or in extracts, such as bacteria, fungi and/or yeasts.

11. The method according to anyone of claims 4 to 10, **characterized in that** it comprises the administration of a composition comprising at least said inactivated bacteria strain in combination with other inactivated bacteria strains.

12. The method according to anyone of claims 4 to 11, **characterized in that** it comprises the administration of a composition comprising at least said inactivated bacteria strain and fertilizers, herbicides, insecticides, fungicides, mineral solutions and/or growing media.

13. The method according to anyone of claims 4 to 12, **characterized in that** said composition is in an appropriate form for soil treatment, treatment of the root part of the plant, treatment of the leaf part of the plant, treatment of the flowering parts, treatment of the fruiting parts and/or treatment of the seed.

14. The method according to anyone of claims 4 to 13, **characterized in that** said composition is present in the form of powder, granules, microgranules, seed treatments, liquid formulations, bacteria encapsulations or liquid suspensions.

Figure 1

Figure 2

Figure 3

Figure 4

Figure 5

Figure 6

Figure 7

Figure 8

Figure 9

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5589381 A
- US 5503652 A
- US 5935839 A
- US 5503651 A
- US 5496547 A
- US 4849008 A
- US 4584274 A
- WO 2003057861 A
- US 6194193 B
- WO 2006071369 A
- US 2012129695 A
- WO 2014046553 A
- WO 2015142185 A
- US 2014109249 A
- US 2014115731 A
- WO 2015142186 A

**Littérature non-brevet citée dans la description**

- **KUCEY.** Plant growth-altering effects of Azospirillum brasilense and Bacillus C-11-25 on two wheat cultivars. *Journal of Applied Microbiology,* 1988, vol. 64 (3), 187-196
- **DUQUENNE et al.** *FEMS Microbiology Ecology,* 1999, vol. 29, 331-339
- **ASADUZZAMAN et al.** Metabolomics Differentiation of Canola Genotypes: Toward an Understanding of Canola Allelochemicals. *Frontiers in Plant Science,* 2015, vol. 5